## Europäisches Patentamt

## European Patent Office

## Office européen des brevets

(19)

(11) Publication number:

# 0 032 513
## B1

(12)

# EUROPEAN PATENT SPECIFICATION

(45) Date of publication of patent specification: **25.04.84**

(51) Int. Cl.³: **A 61 B 10/00**

(21) Application number: **80900270.2**

(22) Date of filing: **30.01.80**

(86) International application number:
**PCT/JP80/00015**

(87) International publication number:
**WO 80/01537 07.08.80 Gazette 80/18**

(54) ULTRASONIC DIAGNOSTIC EQUIPMENT.

(30) Priority: **03.02.79 JP 11587/79**

(43) Date of publication of application:
**29.07.81 Bulletin 81/30**

(45) Publication of the grant of the patent:
**25.04.84 Bulletin 84/17**

(84) Designated Contracting States:
**DE FR GB NL**

(56) References cited:
**FR - A - 2 353 270**
**GB - A - 1 212 349**
**JP - A - 49 025 785**
**JP - A - 51 082 987**
**JP - B - 41 019 959**
**JP - B - 43 029 917**
**JP - B - 45 034 868**
**JP - B - 51 044 773**
**JP - B - 52 047 697**
**US - A - 3 156 110**
**US - A - 4 100 916**
**US - A - 4 167 879**

(73) Proprietor: **FUJITSU LIMITED**
**1015, Kamikodanaka Nakahara-ku**
**Kawasaki-shi Kanagawa 211 (JP)**

(72) Inventor: **MIWA, Hirohide**
**7-10, Miyazaki 6-chome Takatsu-ku**
**Kawasaki-shi Kanagawa 213 (JP)**
Inventor: **SHIMURA, Takaki**
**29-44, Tsurukawa 4-chome**
**Machida-shi Tokyo 194-01 (JP)**

(74) Representative: **Muir, Ian R. et al,**
**HASELTINE LAKE & CO. Hazlitt House 28**
**Southampton Buildings Chancery Lane**
**London WC2A 1AT (GB)**

Courier Press, Leamington Spa, England.

## Ultrasonic diagnostic equipment

This invention relates generally to an ultrasonic diagnostic system according to the precharacterising clause of claim 1, which serves for observing the internal conditions of an object to be measured by emitting ultrasonic waves into the object (such as a human body) and for receiving acoustic waves, resulting from said ultrasonic waves, from inside the object. Such a system is known from US—A—3,156,110.

Ultrasonic diagnostic systems are widely used in order to obtain tomographic images of the internal parts of human bodies. In an ultrasonic diagnostic system, an ultrasonic wave pulse is transmitted into the human body, a resulting echo wave, diffractive wave or transmission wave leaving the body is received and therefrom a tomographic image representative of internal parts of the body is derived. Such ultrasonic diagnostic systems have the merit that they are non-destructive and free of hazards as compared with the use of X-rays.

A widely used ultrasonic diagnostic system utilizes a pulse echo method using an echo wave, and the operation of such an ultrasonic diagnostic system will be explained by way of example. An ultrasonic wave pulse of 1 MHz to 10 MHz is emitted from a probe and transmitted into an object from a probe containing a piezoelectric element and echo signals of a mismatching area are converted into an electrical signal, and location information of said mismatching area can be obtained by displaying the aforementioned electrical signal. (This process is called an A-mode process). A tomographic image can be obtained in such a way that, for scanning, the ultrasonic wave pulse emitting location or angle is sequentially shifted in accordance with tomography and location information of the mismatching areas is displayed combined on the basis of the echo waves of emitted pulses. (This process is called a B-mode process).

In such prior ultrasonic diagnostic systems, the ultrasonic wave pulse emission period is limited to the period required for the echo pulse to be completely received, i.e. the ultrasonic wave pulse is emitted after a period sufficient for the preceding pulse to return as an echo pulse.

Namely, since the velocity of an ultrasonic wave within a human body is above 1500 m/sec, when a maximum diagnostic depth is considered as L m, the period required for a process from pulse emission to reception of echo is 2L/1500 (sec) and therefore the minimum pulse emission period is 2L/1500 (sec).

Thus, the number of scanning lines that can be obtained during one second is limited to 1500/2L (lines). For example, if L=0.2 m, the number of scanning lines becomes 3750, and it is impossible to obtain sufficient scanning lines in a case where a tomographic image is required within a very short period of time, say 0.1 sec, for example.

Such restriction on the scanning lines results in a problem in displaying a tomographic image on an ordinary CRT display unit.

Namely, since a frame speed of about 30 frames/sec is necessary for display of movement within a human body without flickering, the scanning lines of one frame are limited to 125 lines, for example, when L=0.2. The scanning lines of television receivers=512 lines/frame. This means that a tomographic image is displayed very roughly as compared with the television image, curtailing a diagnostic effect.

In other words, the described prior ultrasonic diagnostic system cannot obtain a sufficient number of scanning lines for picking up a dynamic organ such as the heart because of the intrinsic limitation set by the velocity of sound and with such a system the momentary state of an organ and the tomographic image is displayed very roughly. An improvement on such a disadvantageous system is specially desired.

The number of scanning lines can be increased very easily by preparing a plurality of probes containing piezoelectric elements and emitting the ultrasonic waves from these probes simultaneously. Such technology is already disclosed in the Japanese published patent application No. 53-92685. However, when ultrasonic waves of the same frequency are emitted simultaneously from a plurality of probes, it is impossible to distinguish the echo pulses in accordance with probes at the time of reception.

Namely, such prior art has a problem that the acoustic waves mutually interfere because the ultrasonic waves are emitted simultaneously in operation and only an image containing a certain degree of noise can be obtained. Another problem is that the production of a combined display on the screen is difficult because location detection of probes is not carried out.

According to the present invention there is provided an ultrasonic diagnostic system comprising a plurality of electric-acoustic transducers, a transmitting circuit for causing the transducers to transmit separately and substantially simultaneously ultrasonic waves having different detectable characteristics, a receiving circuit containing for each of said different detectable wave characteristics an individual filter which isolates and extracts from received acoustic signals those signals having said particular wave characteristics, and means for providing an acoustic image corresponding to the isolated and extracted signals characterized in that the electric-acoustic transducers are in a plurality of separate probe units which may be separately positioned and directed that the transmitting circuit enables the transducers of the same probe unit to emit ultrasonic waves of the same of said different detectable characteristics, and that the receiving circuit can isolate the received signals relative to each individual probe unit.

With advantage the system incorporates means for detecting the position of the separate probe units relative to each other. An ultrasonic diagnostic system having a detecting mechanism for detecting the locations of a probe is known from FR—A—2,353,270.

In one embodiment of this invention to provide accurate combination of internal image information irrespective of the locations of the plurality of probe units these probe units have mechanical coupling with and detection of secondary dimensional locations of the plural probe units.

For a better understanding of the invention, reference will now be made to the accompanying drawings, in which:

Figure 1 and Figure 2 respectively show the principle of this invention;

Figure 3 shows types of frequencies used in an embodiment of this invention;

Figure 4 shows types of wave width used in an embodiment of this invention;

Figure 5 shows types of waveforms used in an embodiment of this invention;

Figure 6 shows types of pulse trains used in an embodiment of this invention;

Figure 7 shows a block diagram of an embodiment of this invention;

Figure 8 to Figure 10 respectively show block diagrams of embodiments of extraction filters of this invention;

Figure 11 shows another embodiment of this invention;

Figure 12 shows a block diagram of another embodiment of this invention;

Figure 13 shows the construction of a probe used in the embodiment of Fig. 11, and

Figure 14 shows another construction of a probe as used in Fig. 13.

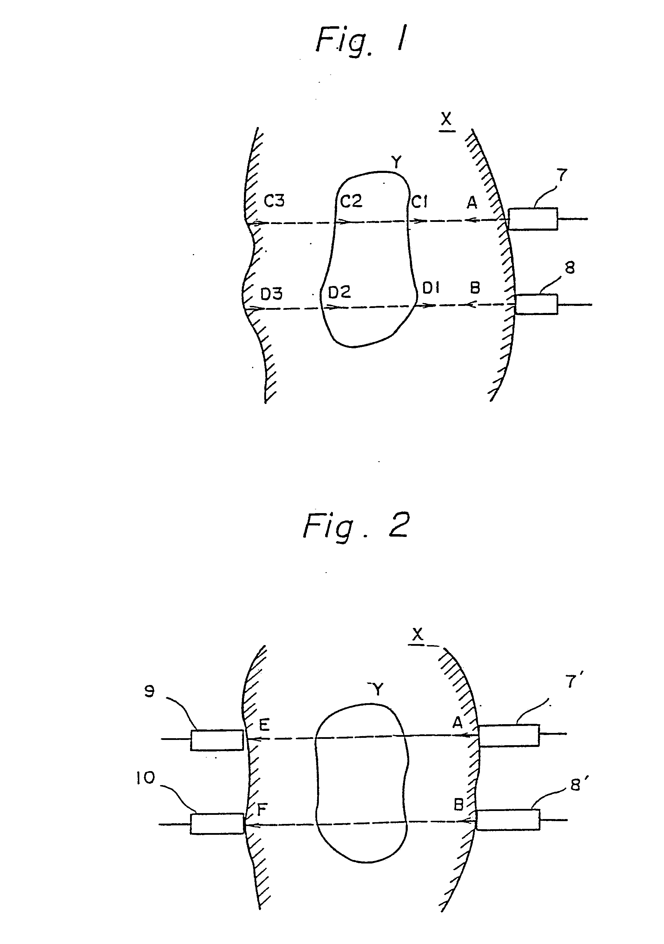

Figure 1 and Figure 2 show a principle of ultrasonic diagnostics employed in this invention. Figure 1 shows an example of echo type and the probes 7, 8 containing an electric-acoustic transducer emit almost simultaneously the ultrasonic waves A, B to a measuring objective. Here, the ultrasonic waves A, B respectively have different characteristics.

The ultrasonic wave A is partly reflected from the area of the objective X where tissue changes. Namely, the echo waves C1 and C2 are returned from the boundary of the organ Y, and the echo wave C3 from the boundary of the objective X. In this same way, the echo waves D1, D2, D3 are returned from the objective X for the ultrasonic wave B.

These echo waves are received by the probes 7, 8 and then converted to electrical signals. However, the probes 7, 8 receive not only the echo waves of their own ultrasonic waves but also those of the ultrasonic waves emitted from the other probe. In other words, the probes 7, 8 receive the echo waves C1 to C3, D1 to D3 and convert them into electrical signals. For this reason, they isolate and extract the component of echo waves C1 to C3 and D1 to D3 from these electrical signals. The time series of extracted echo waves is used as the tomographic information of location of the probes 7, 8.

In the echo type system, the probes 7, 8 may be used for only generation and an additional electric acoustic transducer for reception may be used instead of using the probes 7 and 8 both for generation and reception in common.

For operation in the B-mode, the so-called mechanical linear scanning method where the probes 7, 8 are moved mechanically or the electronic linear scanning mnethod, where a large number of probes 7, 8 are arranged in parallel in the form of array and scanning is carried out electrically, may be employed. It is also possible to apply the sector scanning method which will be described later. The ultrasonic waves A, B are not required to be generated exactly at the same time, and it is enough to generate the ultrasonic wave B before the precedingly generated ultrasonic wave A reaches the maximum diagnostic depth.

Figure 2 shows an example of a transmission type system, where two pairs of probe units 7', 9 and 8', 10 constituting electric-acoustic conversion elements are arranged face to face respectively.

The probe units 7' and 8' generate respectively the ultrasonic waves A and B into the object X. The ultrasonic waves A and B are two kinds of waves having different characteristics.

The transmission wave E obtained when the ultrasonic wave A transmits through the object X, including an organ Y, is received by the probe unit 9 and converted to an electrical signal. Similarly, the transmission wave F obtained when the ultrasonic wave B transmits through the object X, including an organ Y, is received by the probe unit 10 and converted to an electrical signal. The probe units 9 and 10 receive both the transmission waves E and F as in the case of the echo type mentioned above and, therefore, the components of transmission waves E and F are isolated and extracted from electrical signals of the probe units 9 and 10.

In the case of a transmission type system, it is enough to use either of the probe units 9 and 10 as the reception unit. By applying mechanical or electronic linear scanning as in the case of an echo type system, the B-mode process can be employed. Also sector scanning can be applied.

Ultrasonic waves having different characteristics that may be used in this invention will be explained below.

(1) Frequency type using ultrasonic waves of different frequencies as the ultrasonic waves A, B:

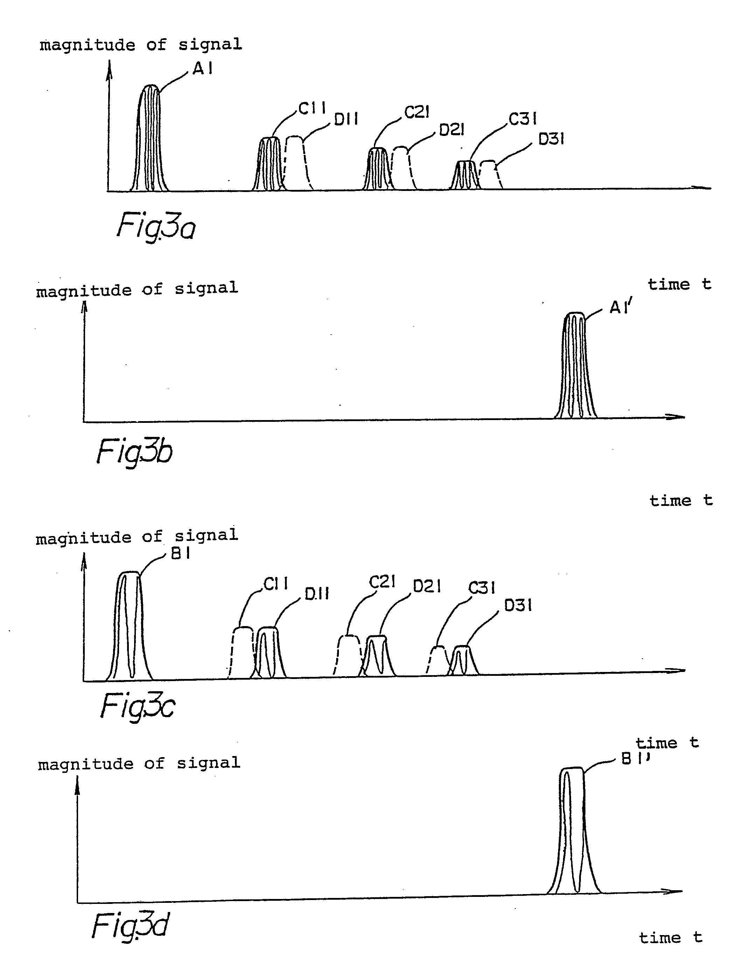

Figure 3 shows an embodiment of the frequency difference type, and in this figure the generated wave and echo wave corresponding to Fig. 1 are indicated on the time axis t.

Namely, the probe 7 generates an ultrasonic wave A1 at a frequency of 2 MHz as shown in Fig. 3(a), while the probe 8 generates an ultrasonic wave B1 at a frequency of 1 MHz as shown in Fig. 3(c). The ultrasonic wave A1 causes the echo waves C11, C21, C31, while the ultrasonic wave B1 causes the echo waves D11, D21, D31, and the received waves of the probe units 7, 8 are respectively the combination of both echo waves as shown in Fig. 3(a), (c) and these combined waves are converted into electrical signals. The received wave in the frequency corresponding to each ultrasonic wave is isolated and extracted from this electrical signal via the band pass filter for the frequency (1 MHz and 2 MHz) corresponding to each ultrasonic wave.

(2) Wave form type using ultrasonic waves in different waveforms as the ultrasonic waves A and B:

This waveform type is classified into (a) waveform width type and (b) waveform shape type.

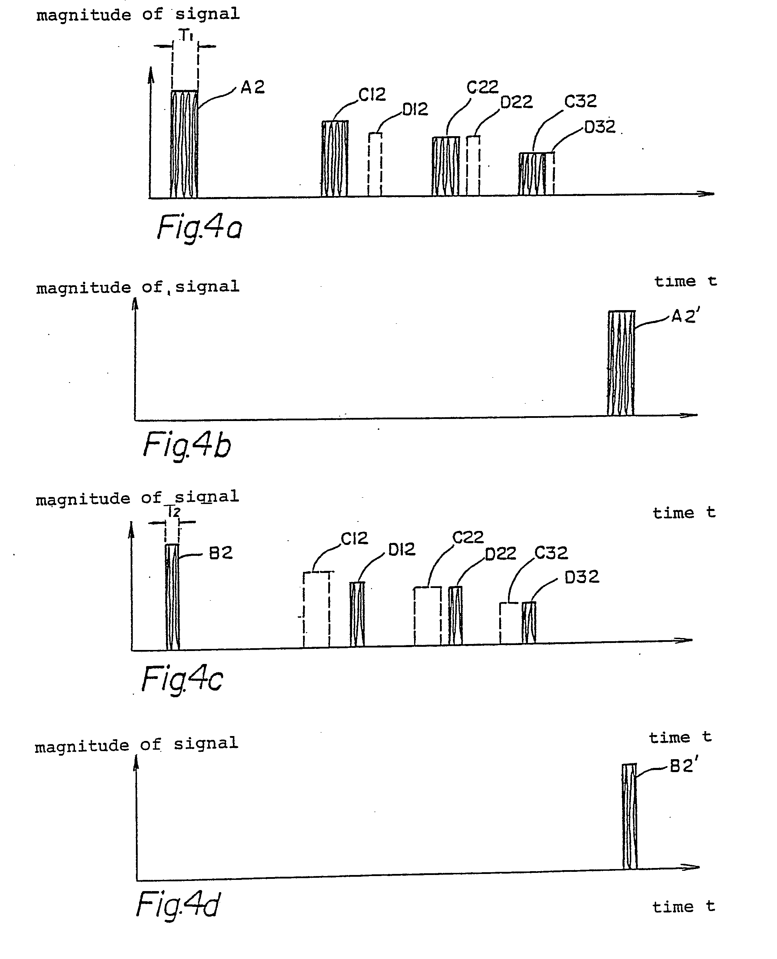

An embodiment of the waveform width type is shown in Fig. 4, where the generated wave and echo wave corresponding to those in Fig. 1 are indicated on the time axis t. The probe unit 7 generates the ultrasonic wave A2 with a width of T1 and frequency of 1 MHz as shown in Fig. 4(a), while the probe unit 8 generates the ultrasonic wave B2 with a width of T2 and frequency of 1 MHz as shown in Fig. 4(c). The ultrasonic wave A2 causes the echo waves C12, C22 and C32 of width T1, while the ultrasonic wave B2 causes the echo waves D12, D22, D32 of width T2, and the received waves of the probe units 7 and 8 is the combination of both echo waves as shown in Figs. 4(a) and 4(c), and these combined waves are converted to electrical signals. The received wave corresponding to each waveform width is isolated and extracted from these electrical signals via a waveform width isolation filter.

For the waveform shape type, waveforms having different gradients or combined waveforms of rectangular and sawtooth waves are used.

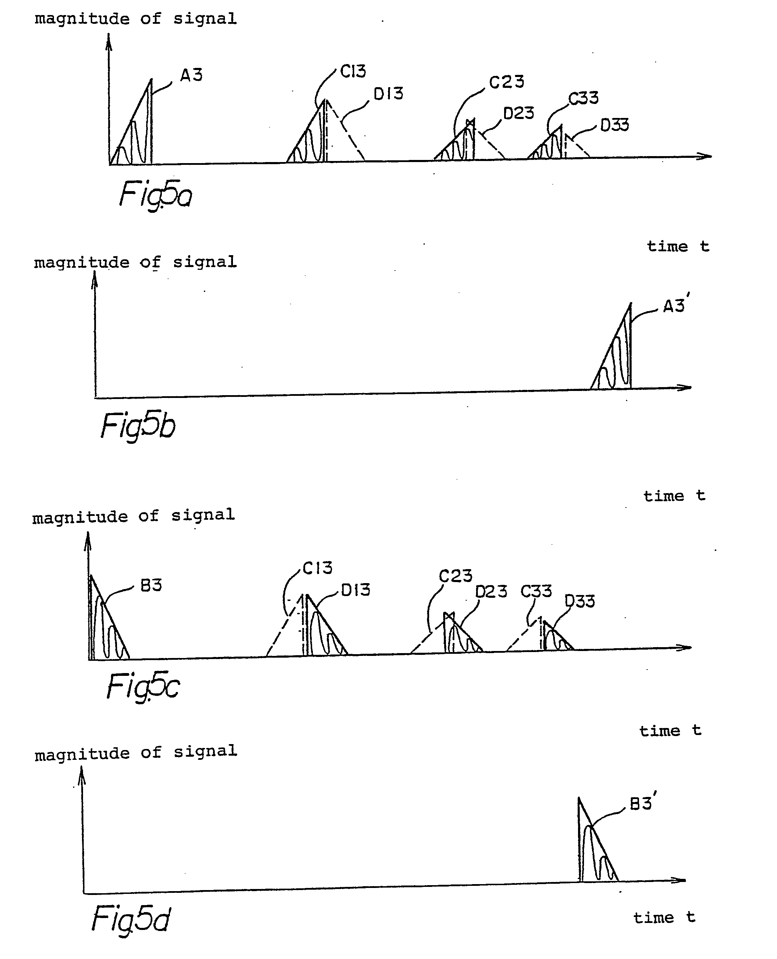

Figure 5 shows an embodiment using waveforms having different gradients, where the generated waves and echo waves corresponding to the system of Fig. 1 are indicated on the time axis t.

The probe unit 7 generates the sawtooth ultrasonic wave A3 having a positive gradient with a frequency of 1 MHz as shown in Fig. 5(a), while the probe unit 8 generates the sawtooth ultrasonic wave B3 having a negative gradient with a frequency of 1 MHz as shown in Fig. 5(c). The ultrasonic wave A3 results in the sawtooth echo waves C13, C23, C33 having the same positive gradient, while the ultrasonic wave B3 results in the sawtooth echo waves D13, D23, D33 having the same negative gradient. The received waveforms of the probe units 7 and 8 are the combinations of the echo waves as is shown in Figs. 5(a) and 5(c) and these waveforms are converted into electrical signals. The received waveform signal corresponding to each sawtooth waveform is isolated and extracted from these electrical signals via a waveform shape isolation filter.

(3) Pulse train type using ultrasonic waves in different pulse trains as the ultrasonic waves A, B:

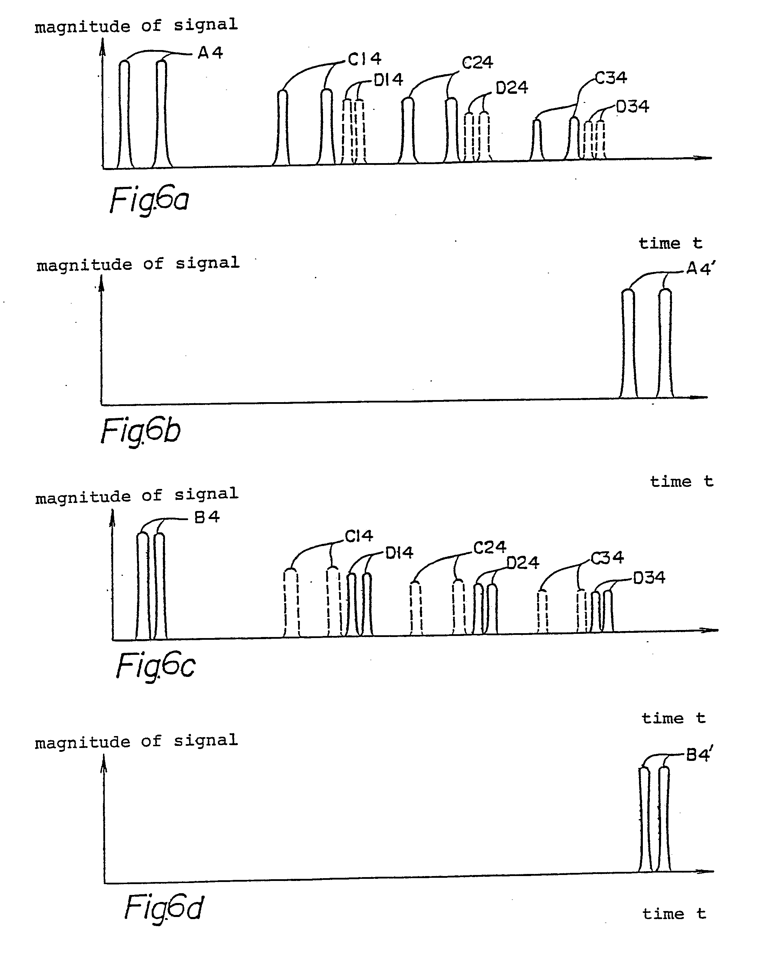

Figure 6 shows an embodiment using pulse trains, where the generated waves and echo waves corresponding to the system of Fig. 1 are indicated on the time axis t.

The probe unit 7 generates the ultrasonic wave pulse train A4 as shown in Fig. 6(a), while the probe unit 8 generates the ultrasonic wave pulse train B4 shown in Fig. 6(c). The ultrasonic wave pulse train A4 results in the echo waves C14, C24, C34 with the same pulse train, while the ultrasonic wave pulse train B4 results in echo waves D14, D24, D34 with the same pulse train. The received waves of the probe units 7 and 8 are the combinations of the echo waves as shown in Fig. 6(a), (c) and these waves are converted into electrical signals. The received wave from signals corresponding to each pulse train arrangement of pulses is isolated and extracted from the combined electrical signals via a pulse arrangement component extraction filter.

The pulse arrangement type also includes those having different pulse widths and different pulse arrangements cr trains.

(4) Combination of a desired two types from the types (1), (2), (3) mentioned above or a combination of more types is also possible.

This invention is explained hereunder in detail by referring to an embodiment.

In an example of an ultrasonic diagnostic system explained below, the echo wave is used as the received wave to be analysed, but it is also possible for a system to use the transmission wave or the diffraction wave as the received wave for analysis.

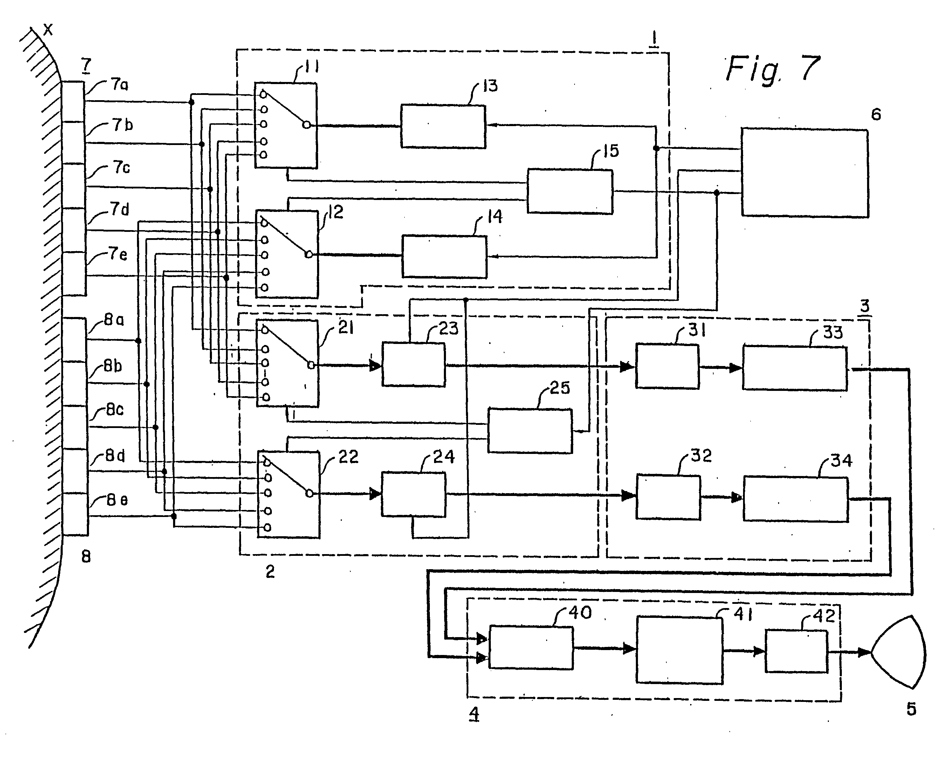

Figure 7 shows the basic block diagram of an embodiment of this invention. In this figure, 1 is a transmission unit; 2 is a receiving gate unit; 3 is a receiving processing unit; 4 is a display control unit; 5 is a display unit; 6 is a control unit; 7 is a first probe unit; 8 is a second probe unit.

The first and second probe units 7 and 8 are array type electric-acoustic transducers of well-known type having, respectively, five probes 7a, 7b, 7c, and 7e; 8a, 8b, 8c, 8d, and 8e.

The array type electric-acoustic transducers use piezoelectric elements which generate ultrasonic waves according to an applied frequency signal or generate electrical signals according to incident echo waves returning from the measured object X.

The transmission unit has first and second transmission selectors 11 and 12. The first transmission selector 11 is associated with the first probe unit 7 and the second transmission selector 12 with the second probe unit 8.

Each of the transmission selectors 11 and 12 has one input terminal and five output terminals, and each of the five output terminals of the first transmission selector 11 is respectively connected to one probe 7a to 7e of the first probe unit 7. Each of the five output terminals of the second transmission selector 12 is connected to one probe 8a to 8e of the second probe unit 8. The input terminals of both transmission selectors 11 and 12 are respectively connected to the first and second signal generator circuits 13 and 14. The input and output terminals of each of the transmission selectors 11 and 12 are connected by means of a rotating arm, which is capable of selectively and sequentially connecting output terminals in accordance with the command issued from the selection circuit 15.

The transmission selectors 11 and 12 are represented by a rotating arm and a contact for ease of understanding, but it is preferable to use a well-known electrical selector circuit having a gate circuit structure.

The selection circuit 15 connects or changes over the transmission selectors 11 and 12 in accordance with a command issued from the control unit 6 and the first and second signal generator circuits 13 and 14 output the signals for generating ultrasonic waves A, B in accordance with a command issued from the control unit 6.

Therefore, the transmission unit 1 outputs a signal for generating the ultrasonic wave A from the first signal generator circuit 13 and a signal for generating the ultrasonic wave B1 from the second signal generator circuit 14. Thus, the signals are sent to the probes 7a to 7e and 8a to 8e that are connected to the selection output terminals of the transmission selectors 11 and 12 in accordance with the command issued from the selection circuit 15.

In Fig. 7, the probes 7a and 8a are selected, and the ultrasonic wave A is generated from the probe 7a and the ultrasonic wave B from the probe 8a.

Next, the transmission selectors 11 and 12 respectively select the second output terminals, in accordance with a command issued from the selection circuit 15, and the first and second signal generator circuits 13 and 14 are connected respectively to the probes 7b and 8b. When a command is issued from the control unit 6, the output signals corresponding to said ultrasonic waves A, B are generated from the first and second signal generator circuits 13 and 14 and thereby the ultrasonic waves A, B are transmitted from the probes 7b, 8b.

Thus, so-called electronic linear scanning is carried out.

The waves A1', B1', A2', B2', A3', B3', A4' and B4' in (b) and (d) of Fig. 3 to Fig. 6 respectively show the relationship of the ultrasonic wave generations and receptions of the probes 7b and 8b for each described waveform type, frequency type and pulse train type.

Ultrasonic wave generation period from the probe 7a to 7b is exactly the same as in the prior art.

As the first and second signal generator circuits 13 and 14, oscillators, oscillating at different frequencies are used for the frequency type, waveform generator circuits generating different waveforms are used for the waveform type, and pulse train generating circuits generating different pulse trains are used for the pulse train type. These circuits are already well known and are not explained here in detail.

The echo waves for the ultrasonic waves transmitted from the transmission unit 1, as explained above, are processed as follows by the receiving gate units and receiving signal processing unit.

The receiving gate unit 2 comprises first and second receiving selectors 21 and 22, and the first receiving selector 21 corresponds to the first probe unit 7, and the second receiving selector 22 to the second probe unit 8.

Each of the receiving selectors 21 and 22 has one output terminal and five input terminals, and each of five input terminals of the first receiving selector 21 is connected to a respective one of the probes 7a to 7e of the first probe unit 7. Each of five input terminals of the second receiving selector 22 is connected to a respective one of the probes 8a to 8e. The output terminals of receiving selectors 21, 22 are connected respectively to the first and second gain compensation circuits 23, 24. The rotating arm being connected with the input terminals of the receiving selectors 21 and 22 selectively and sequentially connects a selected input terminal in accordance with a command issued from the selection circuit 25. The receiving selectors 21 and 22 are illustrated as comprising a rotating arm and contact for ease of understanding, but it is preferable to use a well-known electric selector circuit having a gate circuit structure. The selection circuit 25 connects and changes over the receiving selectors 21 and 22 according to a command issued from the control unit 6.

The gain compensation circuits 23 and 24 are provided for compensating attenuation by changing the gain with time because a reflected wave returning from an area deep in the object is accordingly more attenuated. The gain compensation circuits 23 and 24 change respectively the above-mentioned gain with time according to a command which the control unit 6 sends after generating a command for output of ultrasonic waves.

Therefore, the receiving gate unit 2 connects the signals to the selective input terminals of the receiving selectors 21, 22 in accordance with a command issued from the selection circuit 25.

The electrical signals obtained from the echo waves coming from the probes 7a to 7e and 8a to 8e are input respectively to the gain compensation circuits 23, 24.

In Fig. 7, the probes 7a and 8a are selected and an electrical signal corresponding to the echo wave received by probe 7a is input to the gain compensation circuit 23, while an electrical signal corresponding to the echo wave received by the probe 8a to the gain compensation circuit 24.

The receiving selectors 21 and 22 select sequentially the probes 7b and 8b, 7c and 8c, 7d and 8d, 7e and 8e as in the case of the transmission selectors 11 and 12, and thereby electronic scanning is carried out in synchronization with the selective operation of the transmission selector.

The signals C11 to C31, C12 to C32, C13 to C33, C14 to C34, D11 to D31, D12 to D32, D13 to D33, D14 to D34 of Fig. 3 to Fig. 6 represent the respective electrical signals of the probes 7a to 7e and 8a to 8e for each type of wave.

Outputs of the gain compensation circuits 23 and 24 are input to the receiving signal processing unit 3.

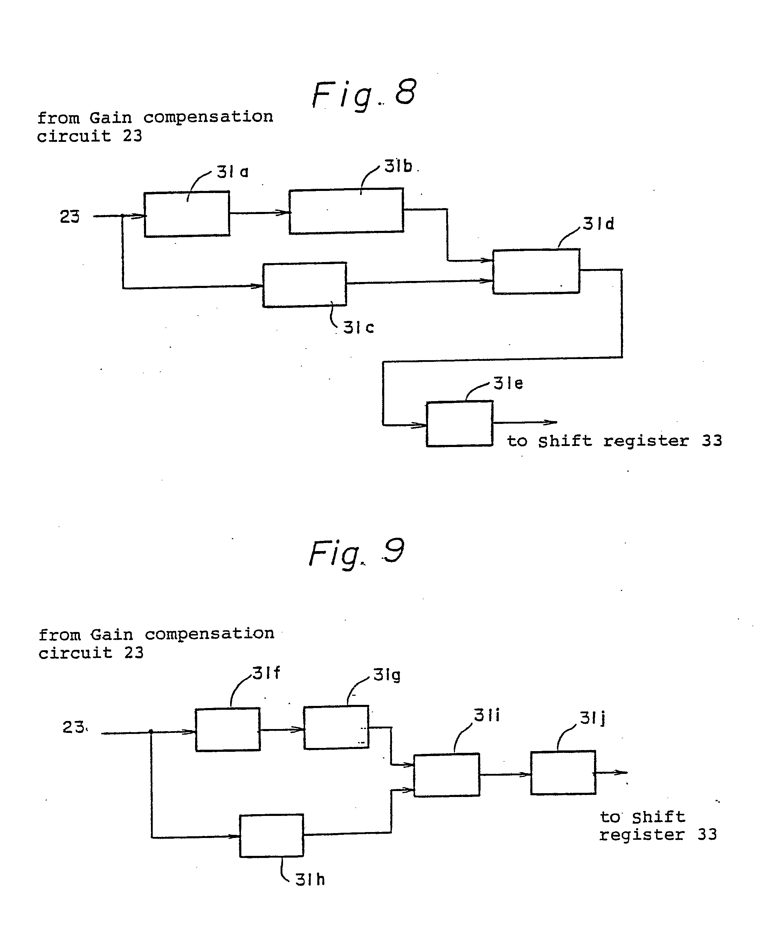

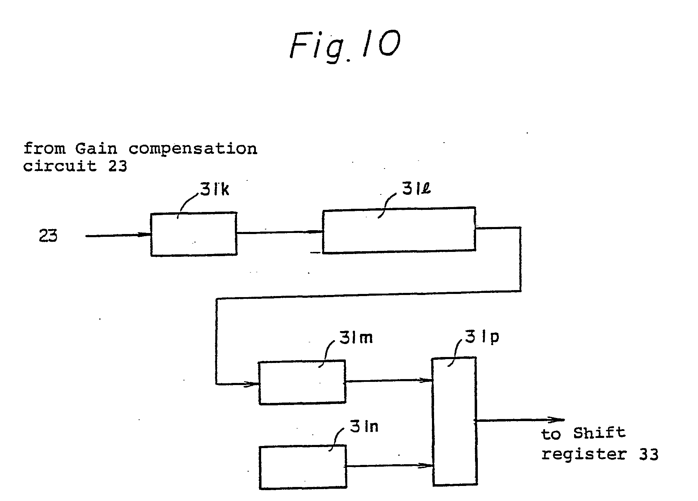

The receiving processing unit 3 comprises first and second extraction filters 31, 32 and first and second shift registers 33, 34. The first extraction filter 31 extracts only the echo wave components corresponding to the generated ultrasonic wave A and then sends it to the shift register 33 after analogue to digital conversion. The second extraction filter 32 extracts only the echo waves corresponding to the generated ultrasonic wave B and sends it to the shift register 34 after analogue to digital conversion. As filters, a band pass filter for each generated ultrasonic wave is used for the frequency type, filters shown in Fig. 8 and Fig. 9 are used for the waveform type, and filters of the kind shown in Fig. 10 are used for the pulse train type.

The information thus stored in the shift registers 33, 34 consists of digital values of echo waves in the time series for the generated ultrasonic waves. The digital values of these time series are displayed on the display unit 5 and they are, therefore, input to the display control unit 4.

The display control unit 4 has a frame memory 41 write circuit 40 and display control circuit 42. The write circuit 40 writes the output signals of shift registers 33, 34 into the corresponding locations of the frame memory 41 in accordance with the locations of probes 7a to 7e, 8a to 8e. The display control circuit 42 sends the storage information of the frame memory 41 at the scanning timing of the display unit 5 and causes a tomographic image to appear on the display unit by displaying echo waves of each time series.

The ultrasonic waves A and B are thus generated, the echo waves are received and a tomographic image is displayed on the display unit by isolating and extracting electrical signals corresponding to the echo waves.

An embodiment of an extraction filter will be explained.

Figure 8 shows a block diagram of a waveform extraction filter.

In Fig. 8, 31a is a waveform width counter; 31b is a waveform width judging circuit; 31c is a delay circuit; 31d is a gate circuit and 31e is an analogue digital conversion circuit.

The operation is explained below. An output signal of the gain compensation circuit 23 is input to the waveform width counter 31a, where the waveform width is counted. For example, in the case of Fig. 4(a), the waveform width counter 31a counts the waveform width of the combined signal of the gain-compensated echo waves C12, D12, C22, D22 and C32 and then outputs it. The waveform width judging circuit 31b selects the width T1 from the counted values and generates a gate signal. Namely, it sends a gate signal of width T1 for the signal C12 and a gate signal of width T1 for the signal C22. The combined signal of C32 and D32 has a width of T1 or more and, therefore, a gate signal of width T1 is also sent since a component of width T1 is also included. Since an output signal of the gain compensation circuit 23 is delayed by the delay circuit 31c and then input to the analogue gate circuit 31d, only the signals C12, C22, C32 are output from the analogue gate circuit 31d. An output of the analogue gate circuit 31d is sampled by the analogue digital conversion circuit 31e at an adequate sampling interval, then analogue-to-digital converted and sent to the shift register 73.

In the same way, the waveform width extraction filter 32 has a similar structure and carries out the same operation, except, only, with the difference that the waveform width judging circuit 31b selects the width T2 and sends a gate signal of width T2, and the gate signal of the width T2 is sent for waveforms having a width of T2 or more than T1.

Figure 9 shows a block diagram of a waveform shape extraction filter which is used as the waveform extraction filter for extracting waveforms of the kind shown in Fig. 5.

In this figure, 31f is a timing generation circuit; 31g is a basic waveform generation circuit; 31h is a delay circuit; 31i is a comparator circuit; and 31j is an analogue-digital conversion circuit.

The operation of this circuit will be explained below. An output signal of the gain compensation circuit 23 is sent to the timing generation circuit 31f. The timing generation signal 31f detects if a signal is not at the zero level and, if not, generates an output pulse at this time. This output pulse is sent to the basic waveform generation circuit 31g, and thereby a basic waveform (A3) which is the same as the generated ultrasonic wave is generated at the timing of this output pulse. This basic waveform is then sent to the comparison circuit 31i.

An output signal of the gain compensation circuit 23 is also fed to the delay circuit 31h, is delayed thereby and then input to the comparison circuit 31i. Therefore, the comparison circuit 31i extracts those waveforms which are the same as the basic waveform from the output of the delay circuit 31h, namely the signals C13, C23, C33 shown in Fig. 5(a), and then sends them to the analogue-digital conversion circuit 31j. The analogue-digital conversion circuit 31j samples them at an adequate sampling interval and sends them to the shift register 33 after analogue to digital conversion.

The waveform shape extraction filter 32 employs a similar structure, with the only difference that the signal B3 as shown in Fig. 5(c) is generated as the basic waveform.

Figure 10 shows a block diagram of a pulse train extraction filter, which extracts pulse trains as shown in Fig. 6.

In this figure, 31k is an analogue-digital conversion circuit; 31 is a shift register; 31m is an auxiliary register; 31n is a basic pattern generation circuit; and 31p is a comparison circuit.

The operation of this filter will be explained below. An output signal of the gain compensation circuit 23 is converted to a digital value by the analogue-digital conversion circuit 31k. The converted digital values are sequentially stored in the shift register 31. The storage data of the shift register 31 is then sent sequentially to the auxiliary register 31m.

For example, if the basic pattern of a signal A4 as shown in Fig. 6(a) comprises 10 bits, then the auxiliary register 31m is also a register of 10 bits. The 10 bits data content of the auxiliary register 31m is compared with the 10 bits basic pattern of the basic pattern generation circuit 31n by the comparison circuit 31p each time the 1 data bit is sent from the shift register 31, and the result of the comparison is sent to the shift register 33.

A pulse train extraction filter 32 has a similar structure, with the only difference that the basic pattern used is the 10-bit pattern of the pulse train B4 shown in Fig. 6(c).

In an embodiment of the above-mentioned electronic linear scanning kind, probes 7 and 8 are constructed so that they can move freely. Therefore, it is possible in the examples shown in Fig. 1, Fig. 2 and Fig. 7 to clearly reproduce the boundary of an organ Y by placing the probes 7, 8 at different angles for said organ Y and by radiating ultrasonic waves from different angles to said organ Y.

The accurate combining and displaying of an acoustic image, based on the generated ultrasonic waves of both probes 7 and 8 that are constructed for free movement, is possible by making use of a probe location detection mechanism, used for an electronic scanning embodiment which will be described later, and resultant combined control technology.

An example of an electronic sector scanning system will now be explained.

As sector scanning systems, both an electronic scanning system and a mechanical scanning system are known.

In an electronic scanning system, plural electric-acoustic transducers are arranged in parallel in the form of an array and are so constructed that ultrasonic waves can be generated at a freely selected desired angle, and moreover, focusing can be effected in such a direction, by changing the generation timing of adjacent electric-acoustic transducers. In the case of a mechanical scanning system, the electric-acoustic transducers are so constructed that the ultrasonic waves can be generated at a freely selected desired angle by rotating the electric-acoustic transducers in the form of the sector around the rotating axis. The sector scanning system is particularly convenient for diagnosis of such areas as cannot be directly diagnosed, for example, the heart at the regions inside the ribs.

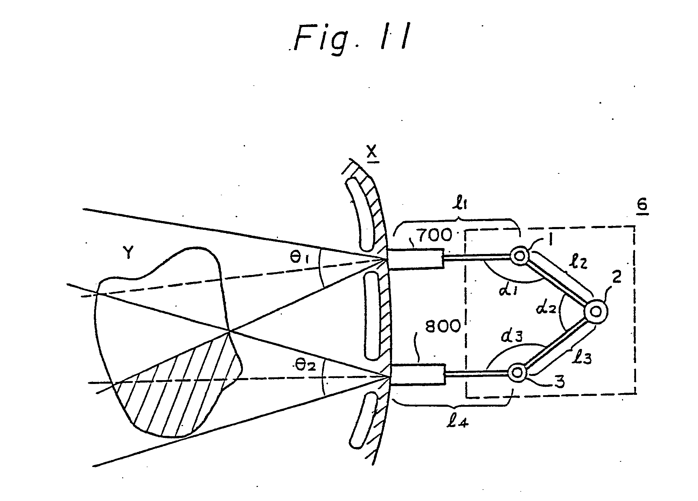

Figure 11 shows an embodiment indicating the principle of an ultrasonic diagnostic system of the electronic sector scanning system kind using the echo pulses (reflected waves) of ultrasonic waves. In this figure, 700, 800 are respectively a first electronic sector scanning probe, and a second electronic sector scanning probe.

In an existing well-known electronic sector scanning type ultrasonic diagnostic system where solely a single electronic sector scanning type probe is used and the generation and reception are sequentially repeated whilst changing the generation angle bit by bit, it is difficult to display a total image of the heart Y within the object X.

In the present invention, the second electronic sector scanning type probe 800 is used in addition to the first electronic sector scanning type probe 700, and thereby a part of the heart which cannot be displayed by the first electronic sector scanning type probe 700 (shown as a hatched area) can be displayed successfully.

In the present invention a multi-beam system using ultrasonic waves having different characteristics is used for the first and second electronic sector scanning type probes 700, 800 for simultaneous scanning by them.

For example, ultrasonic waves having different frequencies or different waveforms may be used.

As an example, toward the most upper angle shown in the figure, an ultrasonic wave of 2 MHz is generated from the first electronic sector scanning type probe 700, while an ultrasonic wave of 3 MHz is generated from the second electronic sector scanning type probe 800.

In this case the generation timing of these ultrasonic waves is the same in order to obtain a display of a moving objective like the heart without any flickering.

As a result, in the case of this embodiment, the echo wave due to mismatching of acoustic impedance of organs inside the measured object X, is received mainly from the part of heart Y by the first electronic sector scanning type probe 700 and the second electronic sector scanning type probe 800. The first electronic sector scanning type probe 700 also receives the ultrasonic wave signal of 3 MHz generated from the second electronic sector scanning type probe 800 in addition to receiving the ultrasonic wave signal of 2 MHz generated by itself, but only the 2 MHz signal is isolated and from probe 700 extracted using a 2 MHz band-pass-filter and thus the picture information of the most upper angle can be obtained.

Then, a transmitting angle of each probe is minutely shifted to the lower angle direction in the figure and similar processing is carried out. By repeating such processing for angles of $\theta_1$, $\theta_2$, the picture information encompassed in the scanning sector angle $\theta_1$ can be obtained from the first electronic sector scanning type probe 700, while the picture information in scanning sector angle $\theta_2$ can be obtained from the second electronic sector scanning type probe 800.

In Figure 11, 6 is a location detector which detects relative location by connecting the first electronic sector scanning type probe 700 and the second electronic sector scanning type probe 800 through, for example, a well-known resolver.

61, 62, 63 are rotating angle converters, which can freely set the optimum angle for diagnosis and take out such angles $\alpha_1$, $\alpha_2$ and $\alpha_3$ as electrical signals respectively. For adequate diagnostic treatment, it is necessary, first of all, to set up the first electronic sector scanning type probe 700 and the second electronic sector scanning type probe 800 to the optimum locations, and the relative location information is then obtained from angular signals corresponding to angles $\alpha_1$, $\alpha_2$, $\alpha_3$ obtained from each rotating angle converter and the fixed rod lengths $l_1$, $l_2$, $l_3$, $l_4$. Namely, the contact points and center directions of the electronic sector scanning type probes 700, 800 and the measured object (specimen) X are determined.

The picture data obtained by said first electronic sector scanning type probe 700 and second electronic sector scanning type probe 800 are location-converted by the relative location information and then combined, and thereby the picture of the total areas covered by the scanning sector angles $\theta_1$ and $\theta_2$ can be displayed.

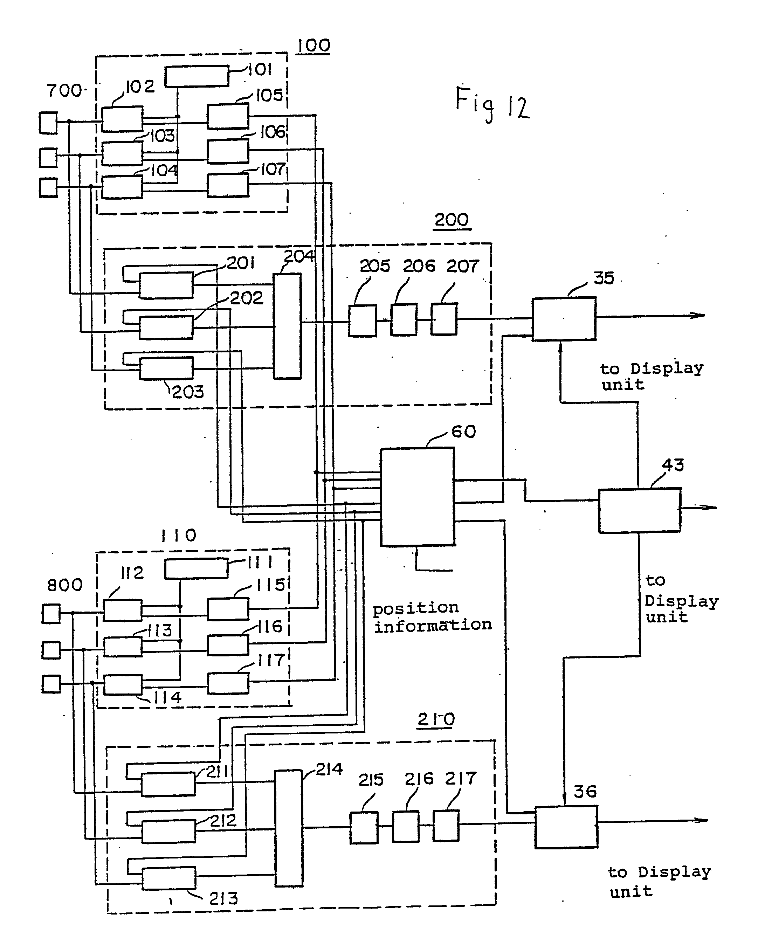

Figure 12 shows a block diagram of an embodiment of this invention. An electronic sector scanning system is shown in this figure and it is a system of the echo wave type. In this figure, 700 and 800 are respectively first and second electronic sector scanning type probes; 100 and 110 are respectively first and second transmission units; 200 and 210 are respectively first and second receiving units; 60 is a control unit; 35 and 36 are respectively first and second memory units; and 43 is a display control unit. The electronic sector scanning type probes 700, 800 are each composed of at least three electric-acoustic transducers. The transmission units 100 and 110 respectively have frequency oscillators 101 and 111 and in said multi-beam system the frequencies of the oscillators 101 and 111 are different. For example, the oscillator 101 oscillates at a frequency of 2 MHz, while the oscillator 111 oscillates at a frequency of 3 MHz. The control unit 60 gives a signal generation command to each of gate signal generating circuits 105, 106, 107, 115, 116 and 117. The gate signals of the gate signal generating circuits 105, 106, 107, 115, 116 and 117 respectively open connected gate circuits 102, 103, 104, 112, 113 and 114, and thereby a frequency signal of the oscillator 101 is given to the electronic sector scanning type probe 700, consisting of three elements, and a frequency signal of the oscillator 111 is given to the electronic sector scanning type probe 800, also consisting of three elements, and thereby the corresponding ultrasonic waves are generated from the electronic sector scanning type probes 700, 800. For electronic sector scanning, it is necessary to generate the gate signals from the gate signal generating circuits 105, 106 and 107 at different timings. Namely, when the gate signals are generated sequentially from the gate signal generating circuits 105, 106 and 107, the ultrasonic wave of the electronic sector scanning type probe 700 advances to the lower left direction. When these signals are generated in the reverse sequence, the ultrasonic wave of the ultrasonic sector scanning type probe 700 advances to the upper left direction. Therefore, the ultrasonic wave is generated by the electronic sector scanning probe 700 in this system in such a way as to effect scanning in the form of a sector, by the changing of the gate signal generation times.

The gate signal generating circuits 115, 116 and 117 also cause the electronic sector scanning probe 800 to generate ultrasonic waves so as to perform a sector scan. Ultrasonic waves of frequency 2 MHz are thus generated from the electronic sector scanning type probe 700, while ultrasonic waves of frequency 3 MHz are generated, sequentially, from the electronic sector scanning type probe 800 in accordance with the required sector scanning. The reflected waves of these ultrasonic waves returning from the measured object X are respectively received by the electronic sector scanning type probes 700 and 800, converted into electrical signals and then input to the receiving units 200, 210, respectively. The receiving units 200, 210 respectively have delay circuits 201, 212, 203 and 211, 212, 213. Each of the delay circuits 201, 202, 203 and 211, 212, 213 is operated by the timing signal of the control unit 60. The control unit 60 gives the timing signals to the delay circuits 201, 202, 203 in the transmission sequence of said transmission gate signals, and also to the delay circuits 211, 212, 213 in the same way. The signals having passed the delay circuits 201, 202, 203 are combined in the adder circuit 204 and then output. Similarly, the signals having passed the delay circuits 211, 212, 213 are combined in the adder circuit 214 and then output. Outputs of adder circuits 204, 214 are input to gain compensation circuits 205, 215, respectively. The gain compensation circuits 205, 215 change the gain with time and compensate for attenuation since reflected waves returning from areas far inside the object X are correspondingly more attenuated. Usually a logarithmic amplifier is used as such gain compensation circuit. The signals thus gain-compensated are input to the band-pass-filters 206, 216. The band-pass-filter 206 allows only signals having the oscillation frequency 2 MHz of the oscillator 101 to pass, while the band-pass-filter 216 allows only signals having the oscillation frequency 3 MHz of the oscillator 111 to pass.

Therefore, the band-pass-filter 206 outputs only the reflected wave component corresponding to the generated ultrasonic wave (2 MHz) of the electronic sector scanning type probe 700, while the band-pass-filter 216 only the reflected wave component corresponding to the generated ultrasonic wave (3 MHz) of the electronic sector scanning type probe 800.

These outputs are input to the analogue-digital conversion circuits 207, 217, respectively, then converted to digital values at the specified sampling rate and input to memory units 35, 36 as picture information.

The address signals corresponding to the sector angles are input to the memory units 35, 36 from the control unit 60, and therefore the digitalized picture information is stored in the addresses corresponding to the sector scanning angles of the memory units 35, 36.

On the other hand, the display control unit 43 receives the coordinates of origin of display screen and sector scanning directions (direction indicated by the dotted line as the centers of angles $\theta_1$, $\theta_2$ in Fig. 11) of the first and second electronic sector scanning type probes 700, 800 based on the location information of the above-mentioned location detector of the control unit 60.

Then the control unit 60 sends a display start command to the display control unit 43. In accordance with such a display start command, the display control unit 43 sequentially sends the electron beam deflection signal according to the sector scanning angle to the display unit using a CRT (not illustrated) and simultaneously sends a synchronization signal for calling up the digitalized picture information corresponding thereto from the memory unit 35 or 36.

The memory unit 35 or 36 which has received such a synchronization signal sequentially sends the digitalized picture information being stored in the address corresponding to the scanning location to the display unit.

The display unit displays a total picture of the heart Y of Fig. 11 on the CRT display screen using the deflection signal thus received for controlling the deflection and scanning of the electron beam and the digitalized picture information for intensity modulation, synchronized to said deflection signal.

At this time, the timing where the picture information is stored in the memory units 33, 34 and the display timing is not always required to be synchronized.

In the above explanation, an electron beam of a CRT is used for sector scanning, but it is also possible to synchronize the sector scanning in accordance with an ordinary television raster by converting the coordinates of the digital data of the memory units 33 and 34. In addition, although, as described, the electronic sector scanning type probes 700, 800 are used in common for both transmission and reception, it is possible to provide a separate electronic sector scanning type probe for reception only.

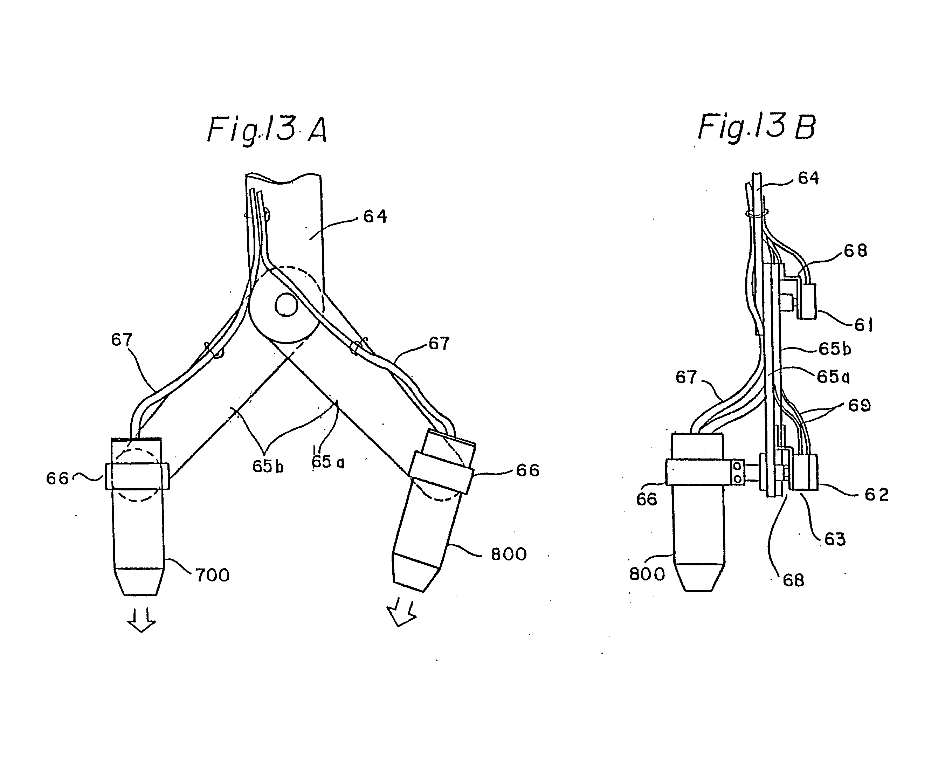

Figure 13 shows the construction of an electronic sector scanning type probe used in Fig. 11; Fig. 13(A) showing the front view and Fig. 13(B) showing a side view.

One arm 65a is engaged with a support arm 65 without freedom of movement, while another arm 65b is engaged with said arm 64 with freedom of rotation.

The rotating angle detector 61 being composed of a potentiometer generates an output corresponding to a rotating angle of the other arm 65b.

The probe 800 is engaged with the end of the one arm 65a with freedom of rotation by means of a metallic probe support 66, and the rotation angle detector 62, composed of a potentiometer, generates an output corresponding to the rotation angle in accordance with the rotation of the probe 800. In the same way, the probe 700 is engaged with the end of the other arm 65b with freedom of rotation by means of a metallic probe support 66, and the rotation angle detector 63, composed of a potentiometer, generates an output corresponding to the rotation angle in accordance with the rotation of the probe 700.

68 is a settling metal, and 67, 69 are connecting cables.

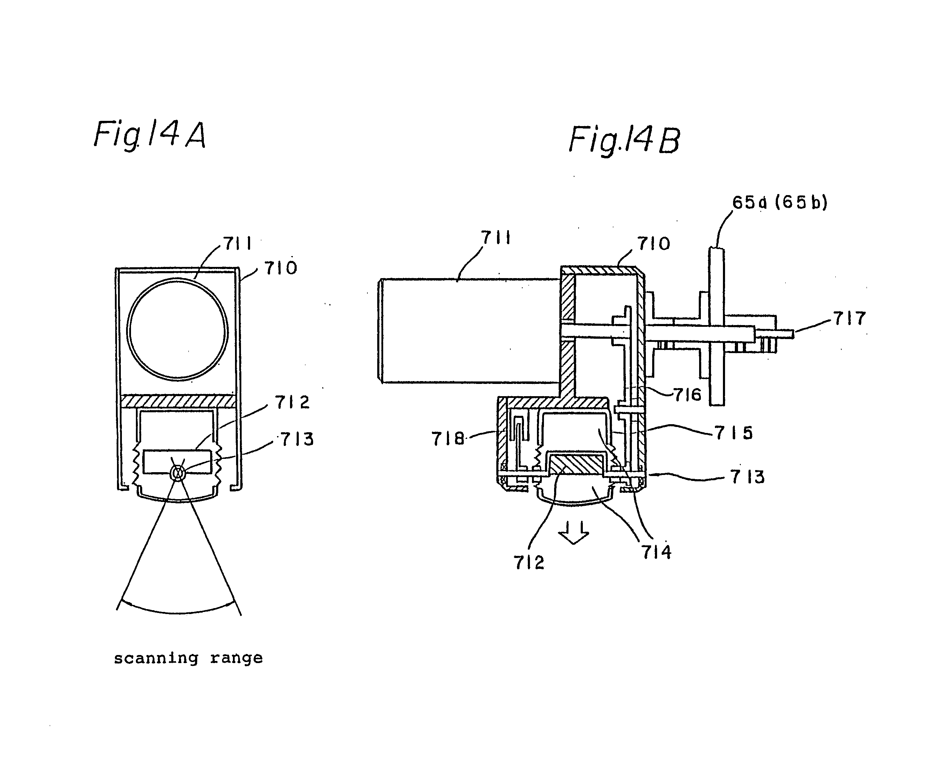

Figure 14 shows the structure of a mechanical sector scanning type probe. Figure 14(a) is a sectional front view and Figure 14(b) is a sectional side view.

In this figure, the probe 710 corresponds to the probes 700, 800 shown in Fig. 13.

An electric-acoustic transducer 712 is accommodated within a variable capacity type vessel 715 and is so constructed that it can rotate around the rotating axis 713. In the vessel 715, the matching liquid 714 for impedance matching is sealed.

The rotating force of a drive motor 711 is transmitted to the rotating axis 713 via the gear 716. Therefore, the transducer 712 can rotate around the rotating axis 713 on account of rotation of the drive motor 711. This rotation is detected by an encoder 718. The probe 710 is coupled to the arm 65b with freedom of rotation and this rotation is transmitted to the rotation angle detector 62, composed of a potentiometer, via the axis 717.

List of reference symbols and items

| | |
|---|---|
| 1 | Transmitting unit |
| 2 | Receiving unit |
| 3 | Receiving processing unit |
| 4 | Display control unit |
| 5 | Display unit |
| 6 | Control unit |
| 7 | Probe unit |
| 7' | Probe unit |
| 700 | Electronic sector scanning type probe |
| 8 | Probe unit |
| 8' | Probe unit |
| 800 | Electronic sector scanning type probe |
| 9 | Probe unit |
| 10 | Probe unit |
| 7a | Electric-acoustic transducer |
| 7b | Electric-acoustic transducer |
| 7c | Electric-acoustic transducer |
| 7d | Electric-acoustic transducer |
| 7e | Electric-acoustic transducer |
| 8a | Electric-acoustic transducer |
| 8b | Electric-acoustic transducer |
| 8c | Electric-acoustic transducer |
| 8d | Electric-acoustic transducer |
| 8e | Electric-acoustic transducer |
| 11 | Transmitting selector |
| 12 | Transmitting selector |
| 13 | Signal generating circuit |
| 14 | Signal generating circuit |
| 15 | Selection circuit |
| 21 | Receiving selector |
| 22 | Receiving selector |
| 23 | Gain compensation circuit |
| 24 | Gain compensation circuit |
| 25 | Selection circuit |
| 31 | Extraction filter |
| 32 | Extraction filter |
| 33 | Shift register |
| 34 | Shift register |
| 40 | Write circuit |
| 41 | Frame memory |

| | |
|---|---|
| 42 | Display control circuit |
| 31a | Waveform width counter |
| 31b | Waveform width judging circuit |
| 31c | Delay circuit |
| 31d | Gate circuit |
| 31e | Analog digital conversion circuit |
| 31f | Timing generation circuit |
| 31g | Basic waveform generation circuit |
| 31h | Delay circuit |
| 31i | Comparison circuit |
| 31j | Analog digital conversion circuit |
| 31k | Analog digital conversion circuit |
| 31l | Shift register |
| 31m | Auxiliary register |
| 31n | Basic pattern generation circuit |
| 31p | Comparison circuit |
| 61 | Rotating angle converter |
| 62 | Rotating angle converter |
| 63 | Rotating angle converter |
| 64 | Support arm |
| 65a | Arm |
| 65b | Arm |
| 66 | Metallic probe support |
| 67 | Connecting cable |
| 68 | Settling metal |
| 69 | Connecting cable |
| 35 | Memory unit |
| 36 | Memory unit |
| 43 | Display control unit |
| 60 | Control unit |
| 100 | Transmitting unit |
| 110 | Transmitting unit |
| 101 | Frequency oscillator |
| 111 | Frequency oscillator |
| 102 | Gate circuit |
| 112 | Gate circuit |
| 103 | Gate circuit |
| 113 | Gate circuit |
| 104 | Gate circuit |
| 114 | Gate circuit |
| 105 | Gate signal generation circuit |
| 115 | Gate signal generation circuit |
| 106 | Gate signal generation circuit |
| 116 | Gate signal generation circuit |
| 107 | Gate signal generation circuit |
| 117 | Gate signal generation circuit |
| 200 | Receiving unit |
| 210 | Receiving unit |
| 201 | Delay circuit |
| 211 | Delay circuit |
| 202 | Delay circuit |
| 212 | Delay circuit |
| 203 | Delay circuit |
| 213 | Delay circuit |
| 204 | Adder circuit |
| 214 | Adder circuit |
| 205 | Gain compensation circuit |
| 215 | Gain compensation circuit |
| 206 | Band-pass-filter |
| 216 | Band-pass-filter |
| 207 | Analog digital convertor |
| 217 | Analog digital convertor |
| 710 | Probe |
| 711 | Drive motor |
| 712 | Electric-acoustic transducer |

713 Rotating axis
714 Matching liquid
715 Variable capacitance type vessel
716 Gear
717 Axis
718 Encoder
A    Ultrasonic wave
B    Ultrasonic wave
C1   Reflected wave
C2   Reflected wave
C3   Reflected wave
D1   Reflected wave
D2   Reflected wave
D3   Reflected wave
E    Through wave
F    Through wave
X    Measuring objective
Y    Organ
Z    Bone
A1   Ultrasonic wave
A2   Ultrasonic wave
A3   Ultrasonic wave
A4   Ultrasonic wave
B1   Ultrasonic wave
B2   Ultrasonic wave
B3   Ultrasonic wave
B4   Ultrasonic wave
A1'   Ultrasonic wave
A2'   Ultrasonic wave
A3'   Ultrasonic wave
A4'   Ultrasonic wave
B1'   Ultrasonic wave
B2'   Ultrasonic wave
B3'   Ultrasonic wave
B4'   Ultrasonic wave
C11   Echo wave
C21   Echo wave
C31   Echo wave
D11   Echo wave
D21   Echo wave
D31   Echo wave
C12   Echo wave
C22   Echo wave
C32   Echo wave
D12   Echo wave
D22   Echo wave
D32   Echo wave
C13   Echo wave
C23   Echo wave
C33   Echo wave
D13   Echo wave
D23   Echo wave
D33   Echo wave
C14   Echo wave
C24   Echo wave
C34   Echo wave
D14   Echo wave
D24   Echo wave
D34   Echo wave

## Claims

1. An ultrasonic diagnostic system comprising a plurality of electric acoustic transducers (7a—7e, 8a—8e), a transmitting circuit (1, 100, 110) for causing the transducers to transmit separately and substantially simultaneously ultrasonic waves having different detectable characteristics (Figs. 3—6), a receiving circuit (2, 3, 200, 210) containing for each of said different detectable wave characteristics an individual filter (31, 32; 206, 216) which isolates and extracts from received acoustic signals those signals having the corresponding wave characteristic, and means (4, 5) for providing an acoustic image corresponding to the isolated and extracted signals characterised in that the electric acoustic transducers are in a plurality of separate probe units (7, 8; 700, 800) which may be separately positioned and directed, that the transmitting circuit (1, 100, 110) enables the transducers of the same probe unit to emit ultrasonic waves of the same detectable characteristic, and that the receiving circuit (2, 3; 200, 210) can isolate the received signals relative to each individual probe unit.

2. An ultrasonic diagnostic system according to claim 1 incorporating means (6 of Fig. 11) for detecting the position of the separate probe units relative to each other.

3. An ultrasonic diagnostic system according to claim 1 and 2 characterised in that the receiving circuit incorporates separate respective memory circuits (35, 36) for each probe unit for storing picture information signals corresponding to the received signals of the probe units and control means (60) which inputs to the memory units (35, 36) signals from the probe position detecting means (6 of Fig. 11) as address signals.

4. An ultrasonic diagnostic system according to any preceding claim incorporating a mechanical coupling mechanism (64, 65a, 65b) for mutually coupling said plurality of probe units 7.

**Patentansprüche**

1. Ultraschall-Diagnoseausrüstung, mit einer Anzahl von elektro-akustischen Wandlern (7a—7e, 8a—8e), einer Übertragungsschaltung (1, 100, 110), welche die Wandler veranlasst, separate und im Wesentlichen simultan Ultraschallwellen zu übertragen, welche verschiedene detektierbare Charakteristiken aufweisen (Fig. 3—6), einer Empfangsschaltung (2, 3, 200, 210), welche für jede der genannten verschiedenen detektierbaren Wellencharakteristiken einen individuellen Filter (31, 32; 206, 216) enthält, der von den empfangenen akustischen Signalen diejenigen isoliert und extrahiert, welche die entsprechende Wellencharakteristik aufweisen, und mit Einrichtungen (4, 5) zur Lieferung eines akustischen Bildes, welches den isolierten und extrahierten Signalen entspricht, dadurch gekennzeichnet, dass die elektrisch-akustischen Wandler in einer Anzahl von separaten Sondeneinheiten (7, 8; 700, 800) sind, welche getrennt positioniert und gerichtet sein können, dass die Übertragungsschaltung (1, 100, 110) die Wandler derselben Sondeneinheit in die Lage versetzt, Ultraschallschwingungen mit derselben detektierbaren Charakteristik zu emittieren, und dass die Empfängerschaltung (2, 3; 200, 210) die empfangenen Signale relativ zu jeder individuellen Sondeneinheit isolieren kann.

2. Ultraschall-Diagnoseausrüstung nach Anspruch 1, mit Einrichtungen (6 der Fig. 11) zum Detektieren der Position der separaten Sondeneinheiten relativ zueinander.

3. Ultraschall-Diagnoseausrüstung nach Anspruch 1 und 2, dadurch gekennzeichnet, dass die Empfängerschaltung für jede Sondeneinheit entsprechende, separate Speicherschaltungen (35, 36) zum Speichern der Bildinformationssignale aufweist, welche den empfangenen Signalen der Sondeneinheiten entsprechen, und eine Steuereinrichtung (60), welche den Speichereinheiten (35, 36) Signale von der Einrichtung (6 in Fig. 11) zum Detektieren der Position der Sonden als Adressensignale zuführen.

4. Ultraschall-Diagnoseausrüstung nach einem der vorhergehenden Ansprüche, gekenn-zeichnet durch einen mechanischen Kupplungs-mechanismus (64, 65a, 65b) zur wechselseitigen Kupplung der genannten Anzahl der Sondeneinheiten (7).

**Revendications**

1. Un système de diagnostic à ultrasons comprenant un ensemble de transducteurs électro-acoustiques (7a—7e, 8a—8e), un circuit d'émission (1, 100, 110) destiné à faire en sorte que les transducteurs émettent séparément et pratiquement simultanément des ondes ultrasonores ayant des caractéristiques détectables différentes (figures 3—6), un circuit de réception (2, 3, 200, 210) contenant, pour chacune des caractéristiques d'onde détectables différentes, un filtre individuel (31, 32; 206, 216) qui isole et extrait des signaux acoustiques reçus les signaux qui ont la caractéristique d'onde particulière correspondante, et des moyens (4, 5) destinés à produire une image acoustique correspondant aux signaux isolés et extraits, caractérisé en ce que les transducteurs électro-acoustiques se trouvent dans un ensemble de sondes séparées (7, 8; 700, 800) qui peuvent être positionnées et dirigées séparément, en ce que le circuit d'émission (1, 100, 110) permet aux transducteurs de la même sonde d'émettre des ondes ultrasonores ayant la même caractéristique détectable, et en ce que le circuit de réception (2, 3; 200, 210) peut isoler les signaux reçus relatifs à chaque sonde individuelle.

2. Un système de diagnostic à ultrasons selon la revendication 1, comprenant des moyens (6 sur la figure 11) pour détecter la position mutuelle des sondes séparées.

3. Un système de diagnostic à ultrasons selon les revendications 1 et 2, caractérisé en ce que le circuit de réception comprend des circuits de mémoire respectifs séparés (35, 36) pour chaque sonde, quit sont destinés à emmagasiner des signaux d'information d'image correspondant aux signaux recus des sondes, et des moyens de commande (60) qui appliquent en entréé, aux unités de mémoire (35, 36), des signaux venant des moyens de détention de positionnement de sonde (6 sur la figure 11) comme signaux d'adresse.

4. Un système de diagnostic à ultrasons selon l'une quelconque des revendications précédentes, comprenant un mécanisme d'accouplement mécanique (64, 65, 65a, 65b) destiné à accoupler mutuellement l'ensemble de sondes (7).

# Fig. 1

# Fig. 2

0 032 513

Fig3a

Fig3b

Fig3c

Fig3d

2

magnitude of signal

T₁

A2

Cl2

C22

D22

C32

D32

*Fig.4a*

time t

magnitude of signal

A2'

*Fig.4b*

magnitude of signal

T2

B2

Cl2

Dl2

C22

D22

C32

D32

*Fig.4c*

time t

magnitude of signal

time t

B2'

*Fig.4d*

time t

3

O 032 513

magnitude of signal

Fig.5a

time t

magnitude of signal

Fig5b

time t

magnitude of signal

Fig.5c

time t

magnitude of signal

Fig.5d

time t

4

magnitude of signal

A4

C14
D14
C24
D24
C34
D34

*Fig.6a*

time t

magnitude of signal

A4'

*Fig.6b*

time t

magnitude of signal

B4

C14
D14
C24
D24
C34
D34

*Fig.6c*

time t

magnitude of signal

B4'

*Fig.6d*

time t

Fig. 7

# Fig. 8

from Gain compensation
circuit 23

# Fig. 9

from Gain compensation
circuit 23

# Fig.10

from Gain compensation
circuit 23

23 → [ 31k ] → [ 31ℓ ]

[ 31m ] → [ 31p ] → to Shift
register 33

[ 31n ] →

# Fig. II

Fig 12

position information

to Display unit

to Display unit

to Display unit

10

## Fig.13 A

## Fig.13 B

0 032 513

# Fig.14A

711
710
712
713

scanning range

# Fig.14B

65d (65b)
711
710
717
716
718
715
713
712
714

0 032 513